# EUROPEAN PATENT APPLICATION

(11) **EP 0 680 945 A2**
(43) Date of publication of application: **08.11.1995**
(21) Application number: 95302977.4
(22) Date of filing: 02.05.1995
(51) Int. Cl.: C07C 229/22, A61K 31/195, A61K 31/14

(54) **Amino acid, carnitine and magnesium supplementation**

(30) Priority: 02.05.1994 ZA 943003; 08.12.1994 ZA 949792; 03.02.1995 ZA 95869
(71) Applicant: OMEARA (PROPRIETARY) LIMITED, Valde Grace, Pretoria (SA)
(72) Inventor: Davis, William Henry, Dunkeld West (ZA); Davis, Henry John, Pretoria (ZA)
(74) Representative: Baverstock, Michael George Douglas

(57) **Abstract**

A novel complex of carnitine and glycine is provided. A composition containing this complex and magnesium chloride and/or L-arginine is also provided. The administration of the complex or composition is found to enhance, synergistically, the absorption of both carnitine and glycine and, when administered with magnesium chloride, to enhance the uptake of magnesium as well. The L-arginine stabilises the complex. The complex and composition have also been found to enhance physical performance in athletes and to promote weight gain and prevent and treat disease in young vertebrate animals.

## Description

### BACKGROUND OF THE INVENTION

THIS invention relates to amino acid, carnitine and magnesium supplementation.

Adenosine triphosphate or ATP, the main energy molecule in plants and animals, is produced by oxidative phosphorylation which is the process whereby energy is released as electrons flow down a potential gradient in the electron transport chain as ATP is synthesized from ADP and Pi. As electrons flow along the chain of carriers, protons are pumped across the inner mitochondrial membrane. The gradient of protons is somehow used to promote the synthesis of ATP and the generally accepted theory relating to the proton flow across the mitochondrial membrane is called the chemiosmotic theory. The rate of electron transport may be measured as the rate of oxygen uptake by mitochondria.

All enzyme-mediated reactions that are known to be catalyzed by ATP have an absolute requirement for magnesium. The reactions involving ATP are so fundamental and widespread that it can be said to influence practically all processes of life.

When an organic phosphate, such as ATP, takes part in a reaction, magnesium is usually its inorganic cofactor, and all participants in reactions known to be dependent on ATP are capable of chelating with magnesium. The stimulation of magnesium uptake, particularly in animals, could therefore be expected to have a beneficial effect in terms of energy utilisation and production.

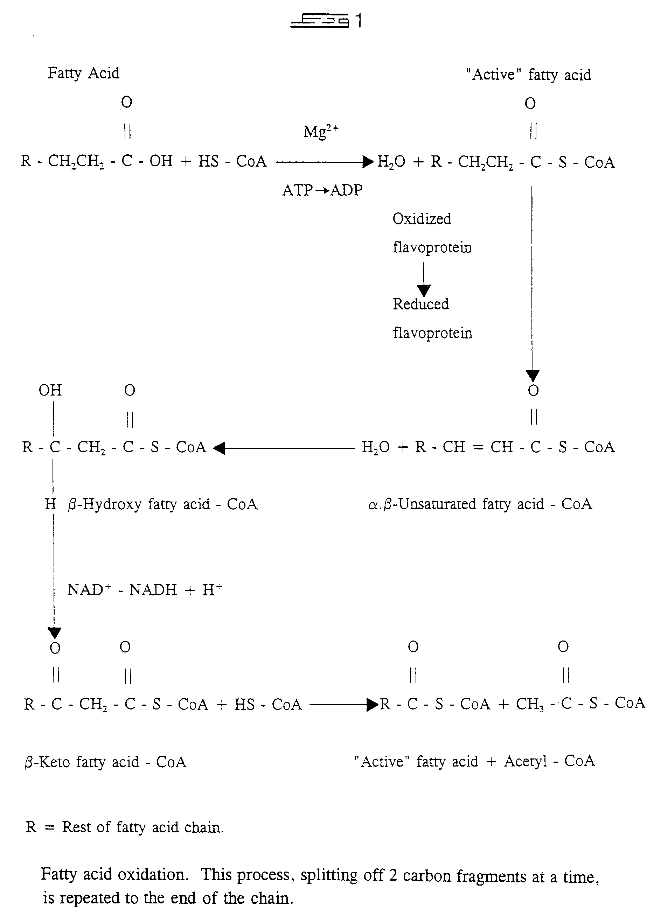

In the animal body, the breakdown or oxidation of fatty acids to acetyl CoA which enters the citric cycle, occurs both inside and outside the mitochondria. Fatty acid oxidation begins with the activation of the fatty acid, as shown in the attached Figure 1, and magnesium and ATP are needed for this. This stimulation of magnesium uptake could therefore also be expected to enhance fatty acid breakdown and energy production.

Active long chain fatty acids formed outside the mitochondria must be linked to carnitine, a lysine derivative, to cross the mitochendrial membrane where the rest of the oxidation occurs and which generates a substantial amount of energy. The presence of carnitine thus stimulates fat oxidation.

Quantitatively, the most important process whereby ATP is generated in muscle tissue under aerobic conditions is glycolation. ATP in muscle and nervous tissue is formed by the transfer of a phosphate group from a phosphogen directly to ADP, such as a phosphorylated guanidine compound, which can be described as an energy rich compound and which is present in these tissues. The phosphagen in vertebrate tissues is usually phosphocreatine and that in invertebrates is usually phosphoarginine.

The first step in the formation of phosphocreatine is the transfer of a guanidino group to glycine with the regeneration of ornithine. The resulting compound, guanidinoacetic acid, is methylated by S-adenosyl-methionine to give creatine. Creatine is then phosphorylated to form phosphocreatine and the transfer of the phosphate group is catalyzed by the enzyme creatine kinase. (The corresponding reaction to produce phospoargenine is catalyzed by arginine kinase.) Both creatine kinase and arginine kinase need the presence of magnesium and ATP to be active.

### SUMMARY OF THE INVENTION

According to the invention a complex of DL-carnitine and/or L-carnitine and glycine which has a mass:charge ratio of 236 when subjected to FAB-MS analysis is provided.

According to another aspect of the invention a composition comprising this complex and magnesium chloride and/or L-arginine monohydrochloride and/or L-arginine is provided.

According to another aspect of the invention a composition comprising a combination of magnesium chloride, L-arginine and/or L-arginine monohydrochloride, glycine and L-carnitine is provided.

The composition may also comprise Vitamin B12, Vitamin C or nicotinamide adenosine dinucleotide (NAD).

According to another aspect of the invention a composition comprising a complex of DL-carnitine and/or L-carnitine and glycine which has a mass:charge ratio of 236 when subjected to FAB-MS analysis and additional L-carnitine, glycine, L-arginine monohydrochloride or L-arginine is provided.

According to another aspect of the invention a method of making a complex of DL-carnitine and/or L-carnitine and glycine which has a mass:charge ratio of 236 when subjected to FAB-MS analysis comprises the steps of:
(i) adding glycine to an amount of DL-carnitine and/or L-carnitine; and
(ii) adding hydrated magnesium chloride to the mixture.

According to another aspect of the invention a method of making a complex of DL-carnitine and/or L-carnitine and glycine which has a mass:charge ratio of 236 when subjected to FAB-MS analysis comprises the steps of:
(i) forming an aqueous solution of DL-carnitine and/or L-carnitine;
(ii) adding glycine to the solution formed in (i); and
(iii) then adding magnesium chloride to the solution.

L-arginine monohydrochloride and/or L-arginine may be added to the solution formed in (ii).

Preferably, the DL-carnitine and/or L-carnitine is completely dissolved before the glycine is added, in step (ii), to the solution formed in (i).

According to another aspect of the invention a method of making a pharmaceutical composition of the invention in the form of an aqueous solution containing L-carnitine, glycine, L-arginine monohydrochloride or L-arginine and magnesium chloride and Vitamin B12 or Vitamin C or nicotinarnide adenosine dinucleotide or additional L-carnitine or additional L-arginine comprises the steps of:
forming an aqueous solution of L-carnitine;
adding glycine to the L-carnitine solution;
adding L-arginine monohydrochloride or L-arginine to the solution;
adding magnesium chloride to the solution; and
mixing the solution for a pre-determined period of time until no further gas is released from the solution and then adding the Vitamin B12 or the Vitamin C or the nicotinamide adenosine dinucleotide or the additional L-carnitine or the additional L-arginine or additional L-glycine.

The complex and compositions of the invention are suitable for use in a method of making a medicament: for use in enhancing carnitine and/or glycine absorption in a vertebrate animal; for use in enhancing magnesium absorption in a vertebrate mammal; for use in enhancing physical performance in a vertebrate animal; for use in promoting growth rate in a young vertebrate animal; and for use in preventing and treating disease in a young vertebrate animal.

The animal is preferably a human, horse, dog, pig, ostrich, turkey, chicken or pigeon.

### DETAILED DESCRIPTION OF THE INVENTION

A novel complex has been formed between glycine and carnitine. It has been found that the complex is relatively easily absorbed by vertebrate animals as compared with uncomplexed carnitine and uncomplexed glycine, which are both normally only poorly absorbed. The complex therefore facilitates the uptake of both carnitine and glycine. Carnitine also has an unpleasant, fishy odour which makes its administration unpleasant. It has, however, been found that the complex of glycine with carnitine has no smell, which makes a composition containing it more palatable.

It has been found that the formation of the complex is facilitated by the presence of magnesium ions, particularly magnesium chloride hexahydrate. If magnesium chloride hexahydrate is present, the complex can be formed in solution or by mixing and then heating the dry ingredients.

Although the complex, when administered alone, results in enhanced uptake of glycine and carnitine, preferred compositions of the invention also contain magnesium chloride or both magnesium chloride and arginine. The magnesium chloride, as described above and below, is added to promote the formation of the complex. However, it is also an important component in its own right in the final composition which is administered to the animal. Its co-administration with the complex has been found not only to enhance the absorption of carnitine and glycine but also to enhance its own absorption which is also normally poor.

Carnitine is known to be unstable in an acidic environment. The formation of a complex with glycine renders it more stable. The presence of arginine, which is a basic amino acid, also buffers the solution containing L-carnitine thereby stabilising the carnitine prior to administration of the composition. The inclusion of arginine, apart from this advantage, is also beneficial in that it is used in the formation of phosphoarginine. Although phosphoarginine is the main phosphagen in invertebrates as outlined above, it is believed it will also stimulate metabolism in vertebrates. Arginine in the composition will therefore also indirectly increase the absorption of carnitine and magnesium chloride. Thus the components in the combination of the invention act synergistically when combined to cross promote their absorption.

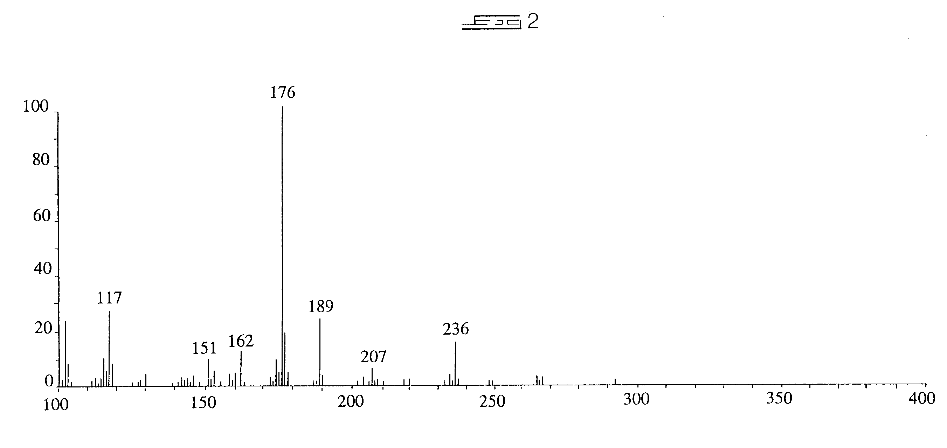

To form the complex an aqueous solution of carnitine was first formed, glycine was then added to the carnitine solution and then arginine was added to the solution and lastly magnesium chloride was added to this solution. The glycine/carnitine complex was detected and was found to have a mass to charge ratio (M/Z) of 236 when the solution was subjected to FAB-MS analysis. The presence of this complex, prepared in solution, is shown in the attached Figures 2 to 5. FAB-MS or fast atom bombardment-mass spectrometry is a soft ionization technique carried out at room temperature which provides the means to detect signals corresponding to intact acylcarnitine molecules. The instrument used in the present invention is a VG 70/70 mass spectrometer iontec saddle field FAB gun. (VG2035 Data system).

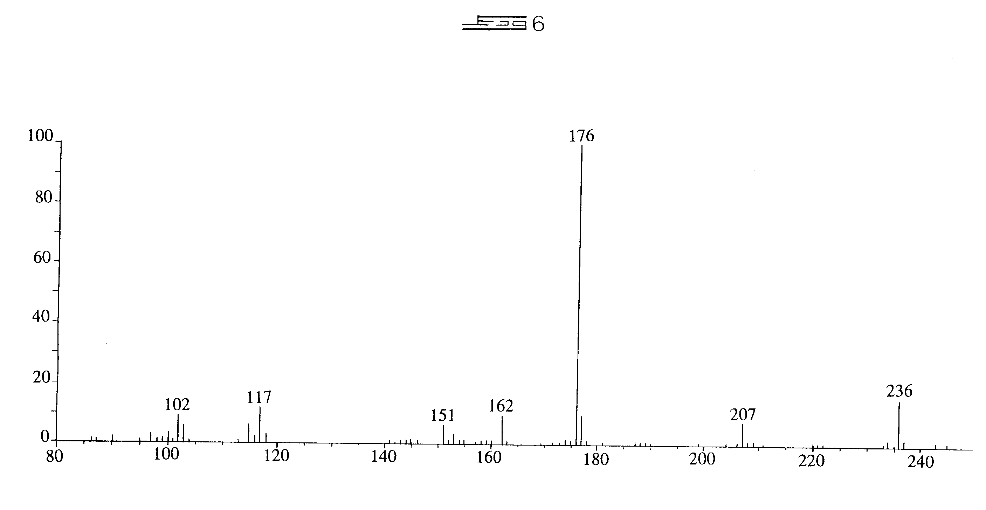

A complex was also found to be formed when arginine was not included and when a dry mix of carnitine, glycine and magnesium chloride hexahydrate was prepared and heated to between 50°C and 70°C. The results of subjecting the resultant composition to FAB-MS analysis are shown in Figure 6. The carnitine/glycine complex is detected at 236 as shown.

In order to verify that the complex produced was in fact a carnitine/glycine complex, an attempt was made to produce a carnitine/glycine complex according to the process of Ziegler et al, a process by which other complexes have been successfully produced. (Ziegler H J, Bruckner P and Binon F (1967), O-Acylation of DL-carnitine chloride. Journal of Organic Chemistry, 32:3989 - 3991.)

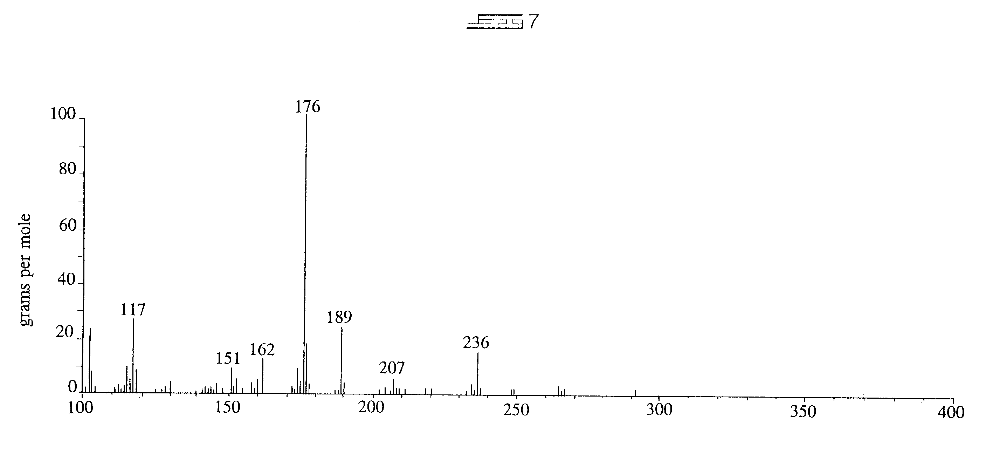

10 mmol of glycine(0.7507 g) and 2.5 mmol of SOCl₂, (0.182 ml) was mixed and the mixture was stirred for 3 hours at a temperature of 80°C. 2.5 mmol of DL-carnitine (0.494 g) in 15 mmol of TCA (2.453 g) at 40°C was then added to the above solution. The temperature was maintained at 80°C for 3 hours. The solution was then poured into dry ether and the precipitate was dissolved in hot isopropyl alcohol and filtered to remove all traces of DL-carnitine chloride. Dry acetone was added to the filtrate and the product subjected to FAB-MS analysis. The results of the FAB-MS analysis are shown in Figure 7. A glycine/carnitine complex was detected at the same value, 236.

The above process of Ziegler et al is time consuming and complicated and produces a number of toxic intermediates. It is therefore not a viable commercial process for producing the complex. However, it does verify that a carnitine/glycine complex is formed by using the method of the invention which requires the presence of magnesium chloride to assist in the formation of the carnitine/glycine complex and the sequential addition of carnitine, glycine and magnesium chloride.

The glycine/carnitine complex of the invention, produced by the method of the invention has the following fonnula:

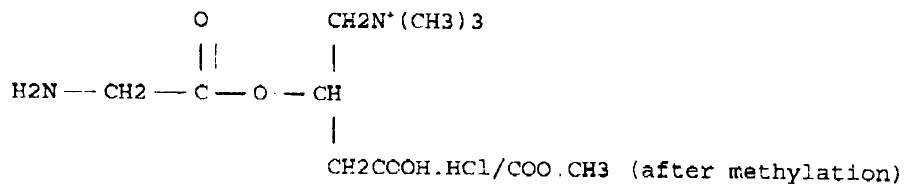

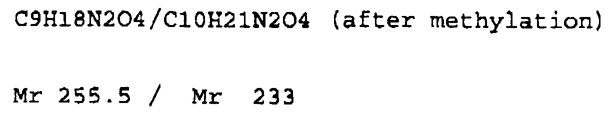

As stated above, the absorption of glycine and carnitine has been found to increase when they are administered in the form of the complex of the invention. In addition, their administration in combination with magnesium chloride and/or arginine has been found to further increase the absorption of each of these components. The increased absorption has been found to have beneficial effects in vertebrate animals. The beneficial effects include increase in energy production in vertebrates, and thereby a stimulation of metabolism, enhancement of physical performance and endurance in long distance human and animal athletes. Administration of the aqueous composition of Example 3 set out below has been found specifically to improve the endurance and stamina of cyclists, tri-athletes and other medium to long distance athletes. It has also been found to improve the performance of animal athletes such as race horses. Increased and enhanced growth rate in young vertebrate animals, such as ostriches, and greater resistance to disease in young animals has also been shown.

Magnesium is also known to decrease stress in pigs. Thus, it is proposed that by administering the complex of the invention to pigs, magnesium absorption will be enhanced and stress and resultant mortality in pigs, particularly pigs being transported to the abattoir, should be reduced.

Without wishing to be bound by theory, it is postulated that the synergistic effect of the components of the composition may be due to some or all of the following:

Magnesium chloride is absorbed in the small intestine and there is evidence to show that if the energy demand in an animal body is not high, not much magnesium chloride is absorbed. While an active working person demands a certain amount of energy and will absorb more magnesium than an inactive person, magnesium absorption often needs to be enhanced in athletes. L-carnitine is also poorly absorbed and it is believed that this is especially so in non-active persons. Only about 15% of L-carnitine administered orally will be absorbed in the small intestine. It is believed that because both increase metabolism, and thus energy production, (L-carnitine facilitates fatty acid oxidation which produces a large amount of ATP and magnesium is needed as a cofactor for ATP and is needed for the initiation of fatty acid oxidation, as described above), an increased demand for the one creates an increased demand for the other, which promotes their absorption. Their co-administration thus cross promotes their absorption;

The complex has been found to stimulate the absorption of carnitine, and thereby increase energy production. The increased absorption of carnitine enhances fatty acid oxidation for which magnesium is required which in turn increases magnesium absorption;

As mentioned above, it is known that glycine is also involved in the initial reaction by which phosphocreatine is eventually formed. By supplementing glycine, therefore, the formation of phosphocreatine is also enhanced. Again without wishing to be bound by theory, it is probable that the complex of the invention is absorbed by a different transport system than uncomplexed carnitine and glycine. It has been shown that dipeptides are better absorbed than single peptides and the glycine/carnitine complex of the invention is probably similarly more easily absorbed.

The compositions of the invention can be formulated in solution and sold in this form, typically in 200ml amber PVC bottles or in sachets containing 5 ml of solution. Alternatively, they can be mixed in dry form with starch and wax to bind the composition and thereby reduce its hygroscopicity. This is then granulated and encapsulated for administration. The dry ingredients can also be tabletted and surrounded by an enteric coating such as that described in South African Patent No. 74/3391, the disclosure in which is incorporated herein by reference, which limits their degradation.

Some examples of compositions of the invention are given below.

### EXAMPLE 1

A typical aqueous composition of the invention comprises:
4% L-carnitine;
1 to 2% glycine;
4% L-arginine monohydrochloride; and
15% magnesium chloride hexahydrate.

A 4% solution of L-carnitine in distilled water was produced and glycine was added to it to form a 1 to 2% solution of glycine. L-arginine monohydrochloride was then added to the solution until a 4% solution of L-arginine monohydrochloride was formed. Lastly, magnesium chloride was added to the solution until a 15% solution was reached. The order of mixing the solution is important to maintain the stability of L-carnitine.

A preservative, possibly vitamin C or ascorbic acid, and typically a 1,5% solution of vitamin C or ascorbic acid, must be added to the composition to limit any microbial and mycotic growth in it. (It is noteworthy that scorbutic animals have 50% less carnitine in heart and skeletal muscle than do controls.)

### EXAMPLE 2

Another aqueous composition of the invention comprises:
5,4% carnitine (100g);
1,6% glycine (30g);
5,4% L-arginine monohydrochloride (100g); and
20,2% magnesium chloride hexahydrate (375g).

A 5,4% solution (100g) of L-carnitine in 1250 ml of distilled water was produced. Glycine was added to it. L-arginine monohydrochloride was then added to this solution. Lastly, magnesium chloride was added. Again the carnitine/glycine complex was formed as shown at 236 in the FAB-MS analysis of Figure 2.

### EXAMPLE 3

Another aqueous composition of the invention comprises:
8,1% L-carnitine (200 g);
2,4% glycine (60g);
8,1% L-arginine monohydrochloride (200g); and
30,5% magnesium chloride hexahydrate (750g).

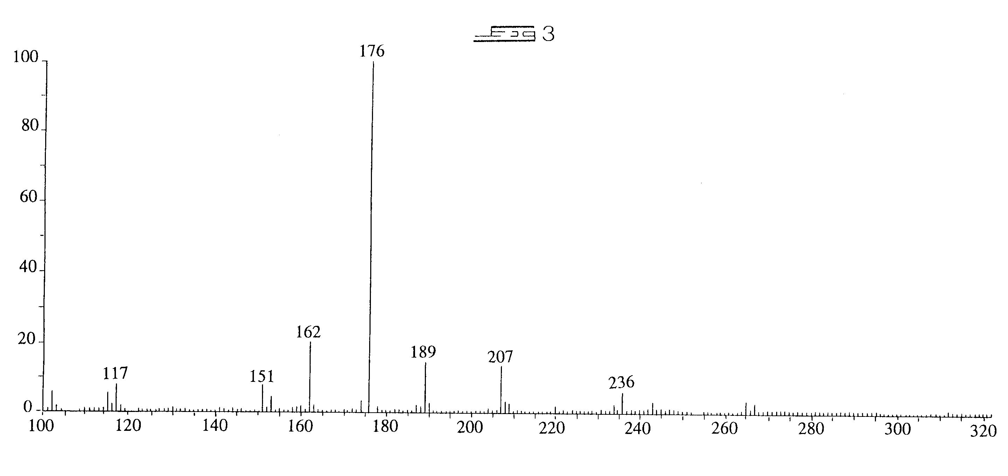

An 8,1% solution of L-carnitine in 1250 ml of distilled water was produced and glycine was added to it. L-arginine monohydrochloride was then added to the solution. Lastly, magnesium chloride was added. The carnitine/glycine complex was formed as shown at 236 in the FAB-MS analysis of Figure 3.

### EXAMPLE 4

Another aqueous composition of the invention comprises:
9,7% L-carnitine (250g);
2,9% glycine (75g);
9.7% L-arginine monohydrochloride (250g);
38,8% magnesium chloride hexahydrate (1000g); and
0.002% Nicotinamide adenine nucleotide (nadide™).

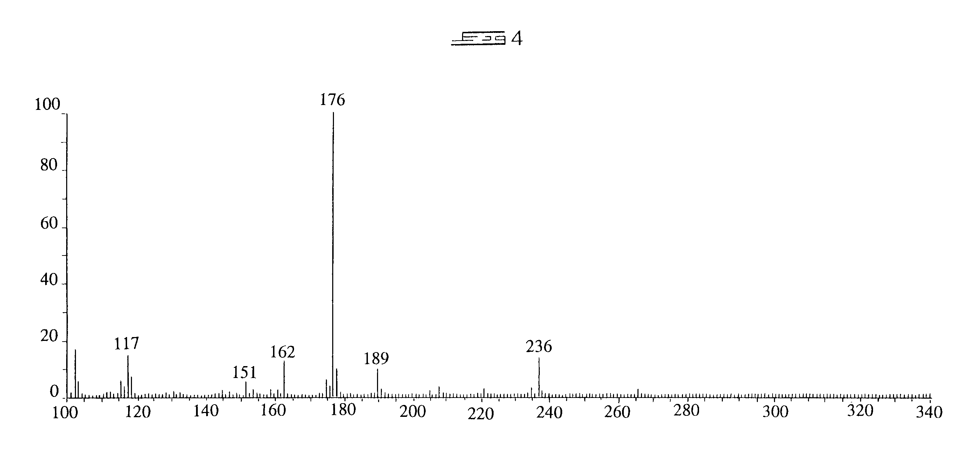

A 9,7% solution of L-carnitine in 1000ml of distilled water was prepared as described for the solutions above. The balance of the components was added and the solution was subjected to FAB-MS analysis. The complex was detected again at 236 as shown in Figure 4 indicating that the addition of NAD do not affect the formulation of the complex.

### EXAMPLE 5

Another aqueous composition of the inventiion comprises:
200g L-carnitine;
60g glycine;
200g L-arginine monohydrochloride;
0.020 g Vitamin B12; and
750g magnesium chloride hexahydrate.

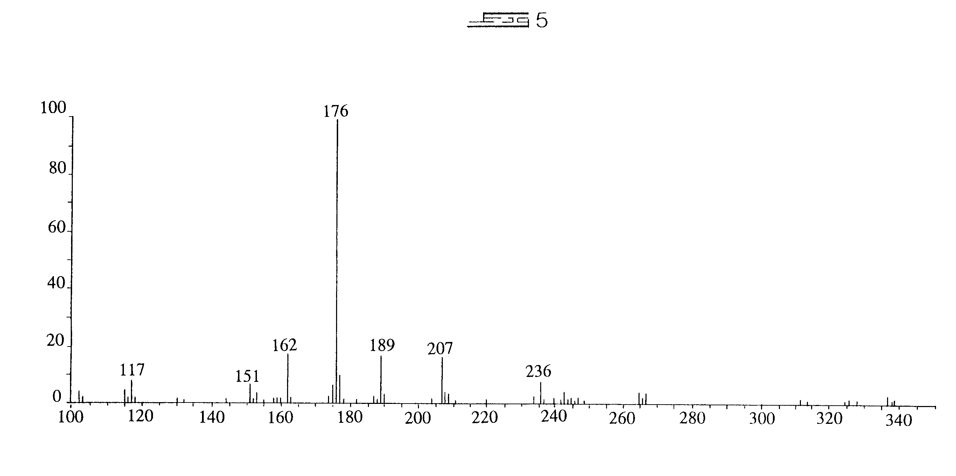

An 0,01% solution of L-carnitine in 1250ml of distilled water was produced. Glycine and arginine and magnesium chloride were then added in the order given for the compositions above and the solution subjected once again to FAB-MS analysis. 0,02g of Vit B12, cyanocoboianiine crystals, was added to 200ml of the solution to give a concentration of 0.01%. The complex was again formed at 236 as shown in Figure 5 indicating that the presence of Vitamin B12 alone did not affect the formation of the complex.

### EXAMPLE 6

Another composition of the invention was prepared by forming a dry mix comprising:
20g L-carnitine
6g glycine;
75g magnesium chloride hexahydrate.

A dry mix of L-carnitine, glycine and magnesium chloride was formed by adding glycine to the L-carnitine and then by adding the magnesium chloride to that mixture. The mixture was heated to between 50 to 70°C. The resulting product was subjected to FAB-MS analysis and the complex was again detected at 236 as shown in Figure 6 indicating that with magnesium chloride present the complex can be formed outside solution.

### EXAMPLE 7

Another aqueous composition of the invention comprising additional L-carnitine, L-arginine and L-glycine and Vitamin B12, Vitamin C and nicotinamide adenoside dinucleotide in a 200ml unit dose comprises the following:

| **COMPONENT** | **CONCENTRATION** |
|---|---|
| L-carnitine | 8.1% |
| Glycine | 2.4% |
| L-arginine | 8.1% |
| Magnesium chloride and | 30,5% |
| Vitamin B12 or | 0.01% |
| Vitamin C or | 7.5% |
| NAD or Nadide | 0,002% |

The addition of Vitamin B12 or Vitamin C or nicotinamide adenosine dinucleotide (NAD) to the composition has been found to enhance its efficacy. Additional L-carnitine and L-arginine and L-glycine, when added, could combine with the already formed first complex to form a second complex which facilitates absorption of the various components, particularly the L-carnitine. This second complex appears to be better absorbed via an alternative absorption pathway to any one of the individual components when not complexed. It has been found to be absorbed through the intestinal wall and also sublingually in humans. It is important, however, when preparing the compositions that the magnesium chloride and/or L-arginine and/or L-carnitine and/or glycine be well mixed, until no further gas is released from the solution, and until a complex between these components forms, before the Vitamin B12 or Vitamin C or nicotinamide adenosine dinucleotide is added to the solution. As the first complex is formed, the solution becomes more viscous.

NAD is used to detoxify alcohol and narcotics addicts and is also believed to relieve some of the symptoms of pulmonary hypertension. A dose of 0.3ml given sublingually 3 times a day would therefore be useful in treating these conditions. NAD also plays a central role in energy metabolism and its inclusion in the composition would therefore be complementary to the action of the other components.

It may be administered orally, sublingually, intravenously or intramuscularly or, particularly for ostriches and chickens, be introduced into drinking water.

The compositions of the invention, apart from their effect in enhancing energy production and availability in athletes, have also been found to be particularly effective in preventing and treating ascitis in chickens and in decreasing the mortality rate amongst ostrich chicks. They have been found to improve their immunity against infection, to enhance growth, to improve their heart function and to prevent hypoglycemia. It is expected that the compositions will have a similar effect in other domestic animals such as pigs, cattle, dogs and cats.

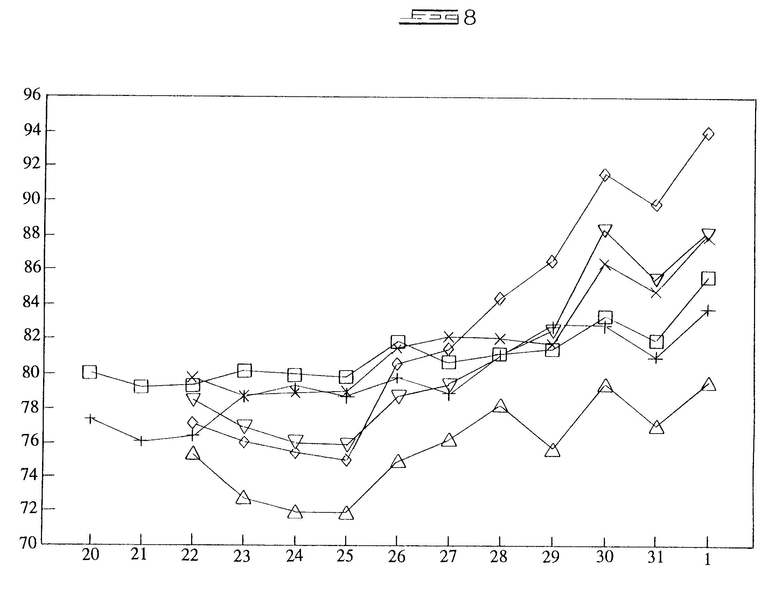

A test was conducted over a month on six groups of sixteen ostrich chicks. The weight gain of the chicks was monitored over this period. One group was given the composition of Example 2 and control groups were each given other formulations set out below. The results are set out in Figure 8.

The graphs indicated by the symbols represent the average weight gain of the groups:
- □: this group was given a food + a multivitamin combination;
- +: this group was given a food + starch, glycine, methionine, folic acid, vitamin B6 and vitamin B12 and magnesium chloride;
- X: this group was given beta carotene, vitamin E, glycine, methionine, starch, magnesium chloride, folic acid, vitamin B6 and vitamin B12;
- ◇: this group was given a food plus the composition of Example 2 (5ml in drinking water per 10 day old ostrich chicks);
- ∇: this group was given beta carotene, vitamin E + arginine, methionine, magnesium chloride, folic acid and vitamin B12;
- △: this group was given beta carotene, vitamin E, methionine, magnesium chloride, folic acid, vitamin B6 and vitamin B12.

The group fed with the composition of the invention initially showed a set back when two of the group, who were diseased, died within the first 3 days. Thereafter, they showed rapid weight gain, outstripping the chicks from other groups.

Another test was performed at VanWyksdorp in the Western Cape, South Africa where four batches of 100 ostrich chicks were raised. The composition of Example 3 was administered to them in a dosage of 15ml in 20 litres of water for the first 14 days of their life. Although no controls were used, an overall 3% mortality was noted. This compared favourably with the average 10 to 25% mortality which is normally encountered.

## Claims

1. A complex of DL-carnitine and/or L-carnitine and glycine characterised in that it has a mass:charge ratio of 236 when subjected to FAB-MS analysis.

2. A composition characterised in that it comprises a complex according to claim 1 and magnesium chloride and/or L-arginine monohydrochloride and/or L-arginine.

3. A method of making a complex according to claim 1 characterised in that it comprises the steps of:
(i) adding glycine to an amount of DL-carnitine and/or L-carnitine; and
(ii) adding hydrated magnesium chloride to the mixture.

4. A method of making a complex according to claim 1 characterised in that it comprises the steps of:
(i) forming an aqueous solution of DL-carnitine and/or L-carnitine;
(ii) adding glycine to the solution formed in (i); and
(iii) then adding magnesium chloride to the solution.

5. A method according to claim 3 or claim 4, characterised in that L-arginine monohydrochloride and/or L-arginine is added to the solution formed in (ii).

6. A composition characterised in that it comprises a combination of magnesium chloride, L-arginine and/or L-arginine monohydrochloride, glycine and L- carnitine.

7. A composition according to claim 2 or claim 6 characterised in that it also comprises Vitamin B12, Vitamin C or nicotinamide adenosine dinucleotide (NAD).

8. Use of a complex according to claim 1 or a composition according to claim 2 or claim 6 or claim 7 in a method of making a medicament for use in enhancing carnitine and/or glycine absorption in a vertebrate animal.

9. Use of a complex according to claim 1 or a composition according to claim 2 or claim 6 or claim 7 in a method of making a medicament for use in enhancing magnesium absorption in a vertebrate animal.

10. Use of a complex according to claim 1 or a composition according to claim 2 or claim 6 or claim 7 in a method of making a medicament for use in enhancing physical performance in a vertebrate animal and/or for use in promoting growth rate in a young vertebrate animal and/or for use in preventing and treating disease in a young vertebrate animal.

11. Use according to claim 8 or claim 9 or claim 10, characterised in that the animal is a human, horse, dog, pig, ostrich, turkey, chicken, or pigeon.
